# EUROPEAN PATENT SPECIFICATION

(11) **EP 1 562 634 B1**
(45) Date of publication and mention of the grant of the patent: **23.08.2006**
(21) Application number: 03789020.9
(22) Date of filing: 10.11.2003
(51) Int. Cl.: A61K 47/48, C07K 14/56

(54) **POSITIONAL ISOMERS OF PEGYLATED INTERFERON ALPHA 2A**
POSITIONELLE ISOMEREN VON PEGYLIERTEM INTERFERON ALPHA 2A
ISOMERES POSITIONNELS D'INTERFERON ALPHA 2A PEGYLE

(30) Priority: 15.11.2002 EP 02025585
(43) Date of publication of application: 17.08.2005
(73) Proprietor: F. HOFFMANN-LA ROCHE AG, 4070 Basel (CH)
(72) Inventor: BRUGGER, Doris, F-68480 Ligsdorf (FR); FOSER, Stefan, 79639 Grenzach-Wyhlen (DE); SCHACHER, Alfred, CH-4125 Riehen (DE); WEYER, Karl, 79415 Bad Bellingen (DE)
(74) Representative: Witte, Hubert
(86) International application number: PCT/EP2003/012498
(87) International publication number: WO 2004/045648

(56) References cited:
- MONKARSH ET AL: "Positional isomers of monopegylated interferon alpha-2a: Isolation, characterization, and biological activity" ANALYTICAL BIOCHEMISTRY, vol. 247, 1997, pages 434-440, XP002916852 ISSN: 0003-2697
- BAILON ET AL: "Rational design of a potent, long-lasting form of interferon: A 40 kDa branched polyethylene glycol-conjugated interferon alpha-2a for the treatment of hepatitis C" BIOCONJUGATE CHEMISTRY, vol. 12, no. 2, March 2001 (2001-03), pages 195-202, XP002264676 ISSN: 1043-1802 cited in the application
- FOSER ET AL: "Isolation, structural characterization, and antiviral activity of positional isomers of monopegylated interferon alpha-2a (PEGASYS)" PROTEIN EXPRESSION AND PURIFICATION, vol. 30, no. 1, July 2003 (2003-07), pages 78-87, XP004432807 ISSN: 1046-5928

## Description

The invention is concerned with positional isomers of monopegylated interferon alpha 2a, with a method for their isolation and for their use in the manufacture of medicaments for the treatment of illnesses, especially for the treatment of viral diseases.

Interferon alpha-2a plays an important role for the treatment of chronic hepatitis C, but it is limited in its efficacy by the short *in vivo* half-life. To improve the half-life and efficacy, interferon alpha-2a was conjugated with a polyethylene glycol moiety. Pegylation changes physicochemical and biological properties of the protein. One effect is the decrease of the proteolytic degradation and the renal clearance. This increases the half-life of the pegylated protein in blood. Another effect is the altered distribution in the body, depending on the size of the PEG moiety of the protein. Interferon alpha 2a pegylated with a large polyethylene glycol moiety (PEG moiety) such as a 40 kDa branched polyethylene moiety 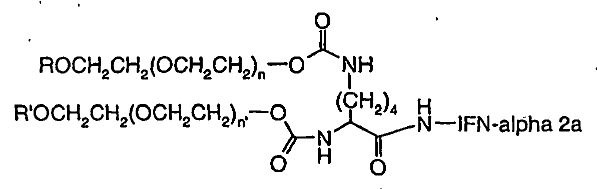 wherein R and R' are independently lower alkyl; n and n' are integers having a sum of from 600 to 1500; and the average molecular weight of the polyethylene glycol units in said conjugate is from about 26,000 daltons to about 66,000 daltons;
has an improved biological activity and exhibits sustained adsorption and reduced renal clearance, resulting in a strong antiviral pressure throughout a once-weekly dosing schedule, see Perry M. C., et al. *Drugs,* 2001,15,2263-2288 and Lamb M. W., et al. *The Annals of Pharmacotherapy,* 2002, 36, 933-938.

See also Monkarsh et al. Analytical Biochemistry, 1997, 247, 434- 440 (Positional Isomers of Mono-pegylated Interferon α-2a) and Bailon et al. Bioconjugate Chemistry, 2001, 12, 195-202 (Rational Design of a Potent, Long-Lasting Form of interferon).

The method for the pegylation of interferon alpha-2a is described in EP A 809 996. Since this pegylation is performed by reaction of PEG2-NHS of formula 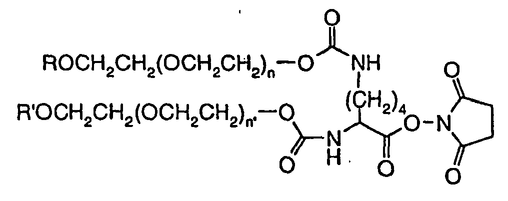 with primary amino groups on for example lysine or to the N-terminus of the interferon alpha.one or more PEG moieties may be attached and form a mixture of unpegylated, mono- and multiple-pegylated interferon. Monopegylated interferon alpha can be isolated from the mixture by methods known in the art. Furthermore, since interferon alpha-2a molecule exhibits 12 sites for pegylation (11 lysines and the N-terminus) it is a mixture of positional isomers. From these possible twelve isomers, nine were isolated and characterized, each of these being conjugated to the branched polyethylene glycol chain at a specific lysine, namely,
at Lys(31) to form interferon alpha 2a pegylated at Lys(31) [referred to as PEG-Lys(31)],
at Lys(49) to form interferon.alpha 2a pegylated at Lys(49) [referred to as PEG-Lys(49)],
at Lys(70) to form interferon alpha 2a pegylated at Lys(70) [referred to as PEG-Lys(70)],
at Lys(83) to form interferon alpha 2a pegylated at Lys(83) [referred to as PEG-Lys(83)],
at Lys(112) to form interferon alpha 2a pegylated at Lys(112) [referred to as PEG-Lys(112)],
at Lys(121) to form interferon alpha 2a pegylated at Lys(121) [referred to as PEG-Lys(121)],
at Lys(131) to form interferon alpha 2a pegylated at Lys(131) [referred to as PEG-Lys(131)],
at Lys(134) to form interferon alpha 2a pegylated at Lys(134) [referred to as PEG-Lys(134)],
at Lys(164) to form interferon alpha 2a pegylated at Lys(164) [referred to as PEG-Lys(164)].

It has been found that PEG-Lys(31) and PEG-Lys(134) have higher activities in an antiviral assay than the mixture, the activity of PEG-Lys(164) was equal to the mixture, whereas the activities of PEG-Lys(49), PEG-Lys(70), PEG-Lys(83), PEG-Lys(112), PEG-Lys(121) and PEG-Lys(131) were lower.

The invention thus is concerned with new positional isomers of pegylated interferon alpha 2a, namely with PEG-Lys(31) and PEG-Lys(134) characterised in that the average molecular weight of the polyethylene glycol moiety (PEG moiety) in said pegylated interferon is from 26,000 daltons to 66,000 daltons, especially of about 40000 daltons.

A chromatography method for the separation of the positional isomers of pegylated interferon alpha 2a based on the local charge differences has been developed. This method consists in a two step separation by ion-exchange chromatography

In a further embodiment the invention is thus concerned with a method for the isolation of the positional isomers of Lys-pegylated interferon alpha 2a which consists in
a) the separation of the positional isomers on a preparative liquid chromatography column with a weak-cation exchange matrix; and
b) the further separation and purification of the fractions from the first step on a preparative column, preferably a HPLC column with a strong-cation exchange matrix, wherein the average molecular weight of the polyethylene glycol moiety (PEG moiety) in said pegylated interferon is from 26,000 daltons to 66,000 daltons.

The separation step a) on the weak-cation exchange matrix was conducted by applying a linear pH gradient from about pH 3,8 to pH 8.0.

The separation step b) was conducted with linear pH gradient of a sodium acetate buffer (A) to a potassium phosphate buffer (B) starting from an initial pH 4.2 to about 4.6, preferably of about pH 4.4, to a final pH of about pH 6.4 to about 6.8, preferably of pH 6.6, said buffer solutions containing in addition up to 12% ethanol and up to 1.5% diethylene glycol, preferably 10% ethanol and 1% diethylene glycol.

The elution of the isomers can be influenced by the initial concentration of the buffer solution. The concentration of the buffer solution is from about 3mM to about 15mM sodium acetate, preferably from about 3 to 7mM, ideally from 3.4mM or 6.8mM.

The separation step b) is carried out at room temperature or at a temperature in the range of about of 27° C to about 35° C, preferably at a temperature of about 30 to 32°C.

This method can also be used analytically for the analysis of the composition of the positional isomers obtained in pegylation reaction of interferon alpha 2a.

The resulting protein samples were collected and analysed by a variety of protein chemical methods such as mass spectrometry peptide mapping, reverse-phase high-performance liquid chromatography (RP-HPLC) peptide mapping, MALDI-TOF spectra of undigested protein, size exclusion HPLC(SE-HPLC) and SDS-PAGE and identified, see examples 2 to 6.

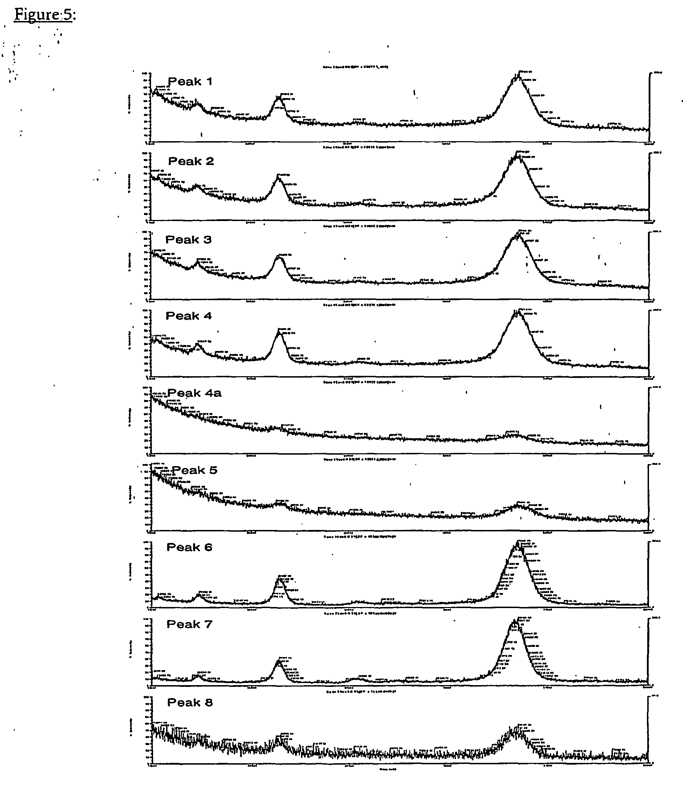

First, the molecular weight of each isomer was determined by MALDI-TOF spectrometry in order to ensure that the pegaylated interferon alpha 2a molecules were still intact after IEC chromatography (Ion Exchange Chromatography) and to confirm the monopegylation. Each IEC peak was measured without further modification. The spectra of all molecules show the expected broad M⁺ peaks with maxima at 63 kDa and the corresponding M²⁺ peaks at 32 kDa and M³⁺ peaks at 21 kDa (Figure 5).

Second, each isomer was proteolytically digested using endo-Lys-C protease and the resulting MALDI-TOF peptide maps were compared with the one derived from the pegylated-interferon alpha 2a reference standard.

Interpretation of the spectra and structural identification of the positional isomers is based on the following considerations:
1. Dipegylation of the isomers can be ruled out because of the molecular weight determination of the entire molecule (see above).
2. The single lysine of a specific isomer having the pegylated polymer group attached is not recognised as lysine by the endo-Lys-C protease (2) *New England Journal of Medicine* 2000, 343,1666-1172. and, therefore, the polypeptide chain is not cleaved at that specific position.
3. It is therefore expected that the peptide map of a specific isomer is lacking the peptides (and only those peptides) which are related to its single pegylated lysine.
4. It is not expected to detect the mass peak of the peptides having the PEG residue attached in the MALDI-TOF peptide maps as the mass range chosen for most accurate detection of the non-pegylated peptides ranges from 850 Da to 6000 Da. The PEG-moiety itself has already a molecular weight of 40 000 Da. However, the pegylated peptides have also been detected using the same digest and trans-3-indoleacrylic acid (IAA) as matrix. For each Lys-C digested isomer a broad peak at 46 - 47kDa was observed, confirming the presence of the monopegylated peptides. Due to the broad mass distribution induced by the PEG-residue, no direct identification of the attached peptides could be made in these experiments (data not shown).

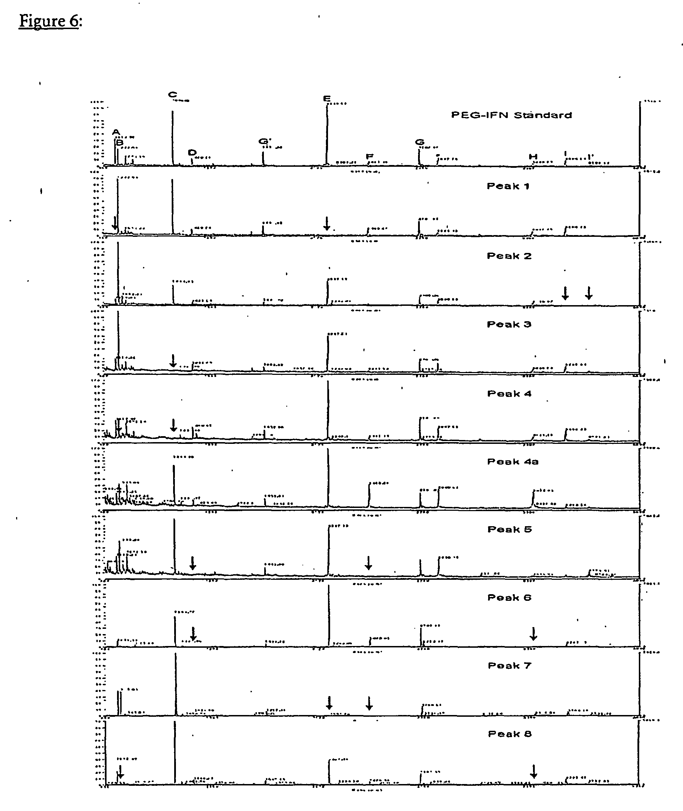

The resulting peptide maps are shown in Figure 6. Peaks that are missing in comparison to the standard are indicated by arrows.

Regarding the spectra of the two references of interferon alpha-2a and pegylated-IFN alpha-2a, no significant differences can be seen. Due to the fact that pegylated-interferon alpha 2a is a mixture of different pegylation isomers, all peptide peaks detected for interferon are detected for pegylated-interferon alpha 2a, too.

In the spectrum of the endo-Lys-C digested protein derived from IEC fraction 1 the peptides comprising amino acids 24 - 31 and 32 - 49 are missing in the region between 850 and 6.000 Da, all other peaks are present. Therefore the PEG residue must be attached to Lys 31.

The other fractions were identified in the same way. In each case the pegylated peptides are missing in comparison to the reference standard spectrum. For fractions 3 and 4a only one peptide peak is missing, for the second peptide 132 - 133 the mass is too small to be detected in the defined mass window. Only fraction 4a could not be identified with this method, no conclusions could be made.

In order to identify isomer 4a, an endo-Lys-C peptide mapping method with RP-HPLC/UV detection has been developed. The protein was digested with endoproteinase Lys-C as described for the MALDI-TOF MS peptide mapping. The peptides were separated by means of a water/acetonitrile/TFA (trifluoro acetic acid) gradient.

With the pegylated-interferon alpha 2a reference standard, 13 peaks were observed. All fractions were collected manually and identified by MALDI-TOF mass spectrometry.

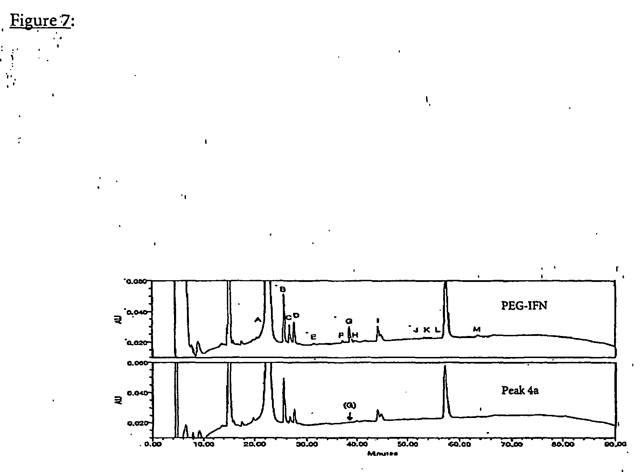

The assignment of the pegylation site of IEC fraction 4a again was done by comparing the chromatogram of the sample to the one obtained for the reference material. The peak containing the two peptides 134 - 164 and 134 - 165 is clearly missing in the sample chromatogram and therefore IEC fraction 4a can be assigned to the isomer containing the PEG at Lys 164. The chromatograms of the pegylated-interferon alpha 2a reference standard (46 µg/mL) and the one of fraction 4a are shown in Figure 7.

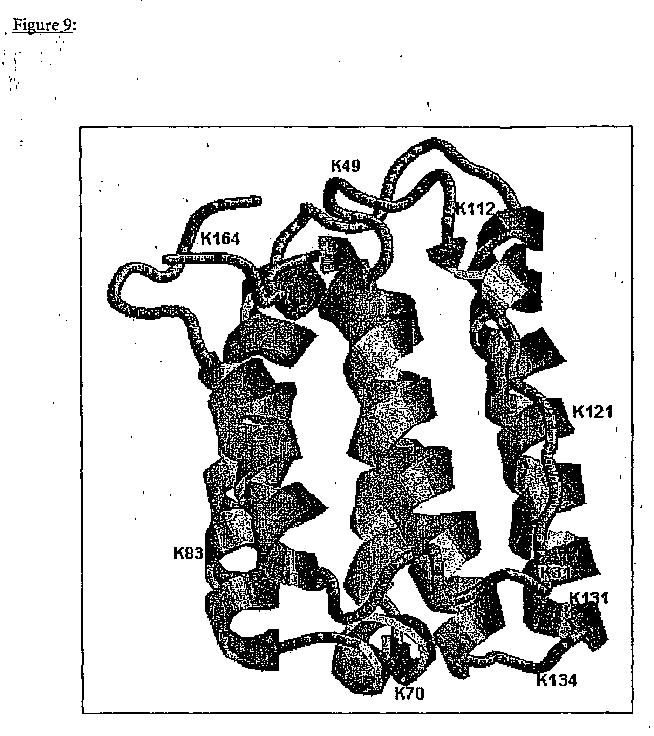

A graphical representation of the 9 pegylated-interferon alpha 2a positional isomers isolated and characterised is given in Figure 9.

The in vitro antiviral activity of the isolated isomers was analysed by the protective effect on Madin-Darby bovine kidney (MDBK) cells against the infection by vesticular stomatitis virus (VSV) and compared with a pegylated-interferon alpha 2a standard according to the procedure described in *J*. *Virol.* 1981, 37, 755-758.

A further embodiment of the invention is therefore use of positional isomers of pegylated interferon alpha-2a molecule, pegylated at Lys(31) and Lys(134), for the preparation of a medicament for antiproliferative, antiviral and immunomodulatory uses. The positional isomers can further be used for the preparation of a medicament for the treatment of viral diseases, especially for the treatment of hepatitis C.

The present invention also comprises the pharmaceutical compositions on the basis of the compounds of formula I or their salts and to methods for producing them.

The pharmaceutical compositions of the present invention used in the control or prevention of illnesses comprises a positional isomer of pegylated IFN alpha 2a, namely PEG-Lys(31) or PEG-Lys(134), and a therapeutically inert, non toxic and therapeutically acceptable carrier material. The pharmaceutical compositions to be used can be formulated and dosed in a fashion consistent with good medical practice taking into consideration the disorder to be treated, the condition of the individual patient, the site of delivery of the positional isomer of pegylated IFN alpha 2a, the method of administration and other factors known to practitioners.

Below the methods and material used in the isolation and the characterisation of the positional isomers of pegylated interferon alpha 2a are described in more detail. The pegylated interferon alpha 2a (PEG-IFN alpha 2a) used for the isolation of the isomers was produced at Hoffmann-La Roche Inc. by the conjugation of lysine ε-amino groups at the surface of the interferon molecule with an activated branched polyethylene glycol moiety of molecular weight 40.000 Da as described in EP A 809996 and in *Bioconjugate Chem.* 2001,12,195-202.

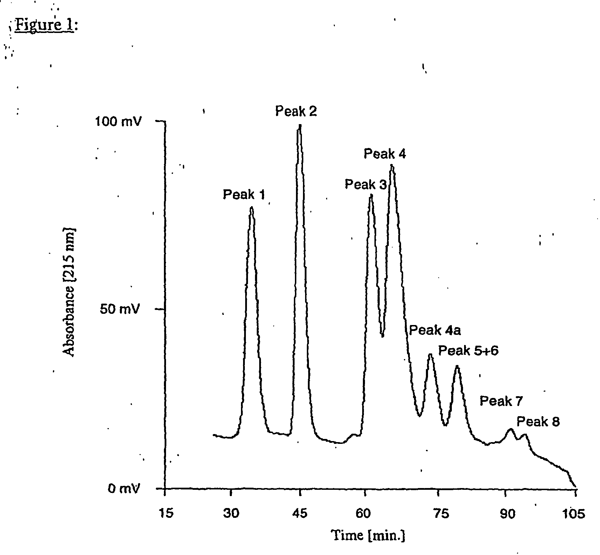

The purity of the samples during the separation of the positional isomers from each purification step was checked using an analytical strong-cation exchange column (TOSOH-BIOSEP, SP-5PW, 10 µm particle size, 7.5 mm diameter, 7.5 cm length). The column was pre-equilibrated with 3.4 mM sodium acetate, 10% ethanol and 1% diethylene glycol, adjusted to pH 4.4 (buffer A). After loading the PEG-IFN samples, the column was washed with buffer A, followed by an ascending linear gradient to 10 mM dibasic potassium phosphate, 10% ethanol and 1% diethylene glycol, adjusted to pH 6.6 (buffer B). The flow rate was 1.0 mL/min and the detection at 218 nm the results are given in Figure 1.

In analogy to the method described above the following analytical method has been found for the analysis of the composition of the positional isomers obtained in pegylation reaction of interferon alpha 2a.

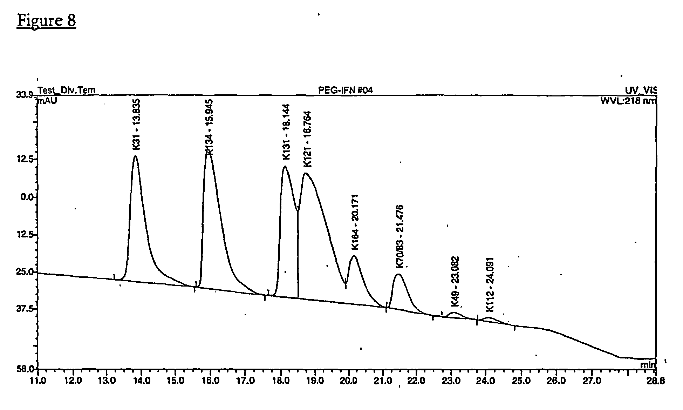

After separation of the monopegylated interferon alpha from the reaction mixture by methods known in the art, the positional isomers are separated by an analytical liquid HPLC (high pressure liquid chromatography) method on a column charged with a strong-cation exchange matrix such as for example nonporous SP-NPR.phase with a particle size of 2.5 µm from, TosoH Bioscience. The mobile phase consist of a buffer A (10% v/v of ethanol; 1% v/v diethylenglycole; 2.3 mM sodium acetate and 5.2 mM acetic acid in purified water; no pH adjustment is made) and a buffer B (10% v/v in ethanol; 1% v/v in diethylenglycole; 16.4 mM KH₂PO₄; and 4.4 mM K₂HPO₄ in purified water, no pH adjustment is made), the results are depicted in Figure 8.

The following examples will further illustrate the invention

### Example 1A Separation of the positional isomers

A two-step isolation and purification scheme was used to prepare the monopegylated isoforms of PEG-interferon alpha 2a.
a) The first step was a separation of the positional isomers on a preparative low pressure liquid chromatography column with a weak-cation exchange matrix (TOSOH-BIOSEP, Toyopearl CM-650S, e.g. Resin Batch no. 82A the diameter of the column being 16 mm, the length 120 cm). A linear pH-gradient of increasing sodium acetate concentration (25 mM, pH 4.0 up 75 mM to pH 7.8) was applied at a flow rate of 0.7 mL/min. Detection was at 280 nm. With this chromatographic step species 1, 2, 5,6 and a mixture of 3, 4, 4a, 7 and 8 could be collected, see Table 1.
b) The fractions were further separated and purified in the second preparation step. A preparative column with the same matrix as the analytical strong-cation exchange column (Resin Batch no. 82A having a ion exchange capacity of 123 mEq/ml) as described above but larger dimensions (30 mm i.d. and 70 mm length), further a higher flow rate and an extended run time was used. As for the analytical method the column was pre-equilibrated with 3.4 mM sodium acetate, 10% ethanol and 1% diethylene glycol, adjusted to pH 4.4 (buffer A). After loading the PEG-IFN samples, the column was washed with buffer A, followed by an ascending linear gradient to 10 mM dibasic potassium phosphate, 10% ethanol and 1% diethylene glycol, adjusted to pH 6.6 (buffer B). The flow rate was 1.0 mL/min and the detection at 218 nm.

The protein concentration of the PEG-IFN alpha 2a isomer was determined by spectrophotometry, based on the 280 nm absorption of the.protein moiety of the PEG-IFN alpha 2a.

An analytical elution profile of 180 µg of PEG-IFN alpha 2a is shown in Figure 1. The result of this method is a separation into 8 peaks, 2 peaks with baseline separation and 6 with partial separation. The decrease of the baseline absorption towards the end of the chromatogram suggests that there were no other monopegylated species of IFN alpha 2a eluting at higher retention time.

In addition, looking carefully at the IEC-chromatogram a further peak close to the detection limit is visible between peaks 2 and 3 indicating the presence of additional positional isomers that should also contribute to the specific activity of the PEG-IFN alpha 2a mixture. Additional species were expected as the interferon alpha-2a molecule exhibits 12 sites for pegylation (11 lysines and the N-terminus). However, given the low abundance of the these species, they were not isolated and characterised.

Isomer samples derived from IEC optimisation runs were investigated directly after the isolation (t = 0) and after 2 of weeks of storage at 5°C (data not shown). No significant differences were observed for the protein derived from IEC-peaks with regard to the protein content as determined by spectrometric methods; nor were any changes to be detected in the monopegylation site, the content of oligo-PEG-IFN alpha 2a, the amount of aggregates and the bioassay activity. Taking into account the relative abundance of the individual isomers - as determined by the IEC method - as well as the specific activities - as determined in the anti-viral assay - almost the total specific bioactivity of the PEG-IFN alpha 2a mixture used for their isolation is recovered (approximately 93%).

The analytical IE-HPLC was used to check the purity of the individual isomers with respect to contamination with other positional isomers in the IEC fractions. The peaks 2, 3, 4, 4a, 5 and 7 had more than 98%, the peaks 1 and 8 had 93% and peak 6 had 88 % purity.

**Table 1:**

| PEG-peptides identified by comparison of the Lys-C digest spectra of the isomers and the reference standard. | | | |
|---|---|---|---|
| Identified PEG Sites in the separated PEG-IFN Species | | | |
| Peak | | missing peaks in peptide map | |
| PEG-IFN | PEG site | Mᵣ (DA) | Sequence |
| Peak 1 | K³¹ | A,E | 24-49 |
| Peak 2 | K¹³⁴ | I, I' | 134-164 |
| Peak 3 | K¹³¹ | C | 122-131^{a} |
| Peak 4 | K¹²¹ | B, C | 113-131 |
| Peak 4a | K¹⁶⁴ | ^{b} | 134-164^{a,b} |
| Peak 5 | K⁷⁰ | D, F | 50-83 |
| Peak 6 | K⁸³ | D, H | 71-112 |
| Peak 7 | K⁴⁹ | E, F | 32-70 |
| Peak 8 | K¹¹² | B, H | 84-121 |

| | | | |
|---|---|---|---|
| ^{a}132-133 too small to detect. | | | |
| ^{a,b} RP-HPLC. | | | |

The fractions were characterised by the methods described in examples 2 to 6.

### Example 1B Analytical separation of positional isomers of mono-pegylated interferon alpha 2a

| | | |
|---|---|---|
| HPLC Equipment: | HP1100 | |
| Column: | SP-NPR, TosoH Bioscience, Particle size: 2.5µm, nonporous, Order#: 13076 | |
| Injection: | 5-10 µg monopegylated IFN | |
| mobile Phase: | *Buffer A:* | |
| | 10% v/v | Ethanol |
| | 1% v/v | Diethylenglycol |
| | 2.3 mM | Na-Acetat |
| | 5.2 mM | Acetic acid, in purified water, no pH adjustment |
| | *Buffer B:* | |
| | 10% v/v | Ethanol |
| | 1% v/v | Diethylenglycol |
| | 16.4 mM | KH₂PO₄ |
| | 4.4 mM | K₂HPO₄, in purified water, no pH adjustment |
| Gradient: | 0 Min | 40 %B |
| | 2 Min | 40 %B |
| | 2.1 Min | 48 %B |
| | 25 Min | 68 %B |
| | 27 Min | 75 %B |
| | 30 Min | 75 %B |
| | 34 Min | 40 %B |
| | 40 Min | 40 %B |
| Flow: | 1.0 ml/min | |
| Column Temperature: | 25°C | |
| Detection: | 218 nm | |

a typical Chromatogram is given in Figure 8.

### Example 2 Analysis of the fractions by mass spectrometry peptide mapping

Mass spectra were recorded on a MALDI-TOF MS instrument (PerSeptive Biosystems Voyager-DE STR with delayed extraction). Each IEC fraction (Ion Exchange Chromatography) was desalted by dialysis, reduced with 0.02 M 1,4-dithio-DL-threitol (DTT) and alkylated with 0.2 M 4-vinyl pyridine. Then the proteins were digested with endoproteinase Lys-C (Wako Biochemicals) in 0.25 M Tris (tris(hydroxymethyl)-aminoethane) at pH 8.5 with an approximate enzyme to protein ratio of 1:30. The reaction was carried out over night at 37 °C.

A solution of 20 mg/ml α-cyano-4-hydroxycinnamic acid and 12 mg/ml nitrocellulose in acetone/isopropanol 40/60 (v/v) was used as matrix (thick-layer application). First, 0.5 µL of matrix was placed on the target and allowed to dry. Then, 1.0 µL of sample was added. The spectra were obtained in linear positive ionisation mode with an accelerating voltage of 20.000 V and a grid voltage of 95 %. At least 190 laser shots covering the complete spot were accumulated for each spectrum. Des-Arg¹-bradykinin and bovine insulin were used for internal calibration.

### Example 3 high-performance liquid chromatography (RP-HPLC) Peptide Mapping

The peptides were characterized by reverse-phase high-performance liquid chromatography (RP-HPLC) Peptide Mapping. The IEC fractions were reduced, alkylated and digested with endoproteinase Lys-C as described for the MALDI-TOF MS peptide mapping. The analysis of the digested isomers was carried out on a Waters Alliance HPLC system with a Vydac RP-C18 analytical column (5 µm, 2.1 × 250 mm) and a precolumn with the same packing material. Elution was performed with an acetonitrile gradient from 1 % to 95 % for 105 min in water with a flow rate of 0.2 mL/min. Both solvents contained 0.1 % (v/v) TFA. 100 µL of each digested sample were injected and monitored at 215 nm.

### Example 4 MALDI-TOF spectra of undigested protein

An 18 mg/ml solution of trans-3-indoleacrylic acid in acetonitrile/0.1 % trifluoroacetic acid 70/30 (v/v) was premixed with the same volume of sample solution. Then 1.0 µL of the mixture was applied to the target surface. Typically 150 - 200 laser shots were averaged in linear positive ionisation mode. The accelerating voltage was set to 25.000 V and the grid voltage to 90 %. Bovine albumin M⁺ and M²⁺ were used for external calibration.

### Example 5 SE-HPLC (size exclusion HPLC)

SE-HPLC was performed with a Waters Alliance 2690 HPLC system equipped with a TosoHaas TSK gel G 4000 SWXL column (7.8 × 300 mm). Proteins were eluted using a mobile phase containing 0.02 M NaH₂PO₄, 0.15 M NaCl, 1% (v/v) diethylene glycol and 10 % (v/v) ethanol (pH 6.8) at a flow rate of 0.4 mL/min and detected at 210 nm. The injection amounts were 20 µg of each isomers.

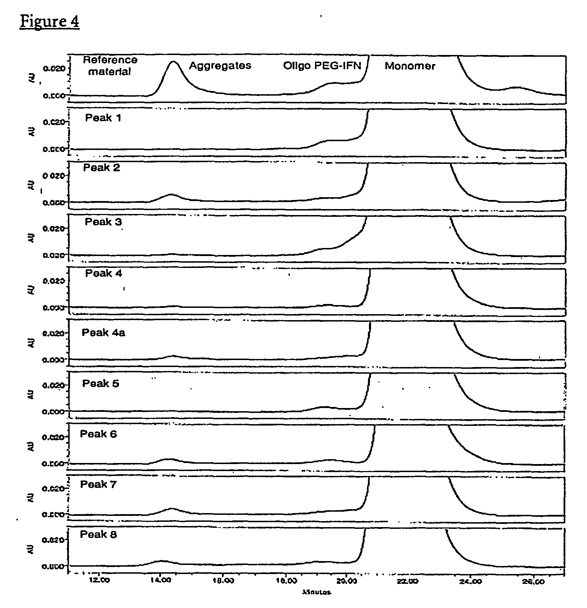

Size Exclusion HPLC and SDS-PAGE were used to determine the amount of oligo-PEG-IFN alpha 2a forms and aggregates in the different IEC fractions. The reference material contains 2.3 % aggregates and 2.2 % oligomers (Figure 4).

Peaks 1, 4, 4a, 5, 6 and 8 contain < 0.7 % of the oligopegylated IFN alpha 2a forms, whereas in,peaks 2, 3, and 7 the percentage of the oligopegylated IFN alpha 2a forms are under the detection limit (< 0.2 %). In the case of the aggregates a different trend could be seen. In all peaks the amount of aggregates is below 0.9 %.

### Example 6 SDS-PAGE

SDS-PAGE was carried out both under non-reducing and under reducing conditions using Tris-Glycine gels of 16 % (1.5 mm, 10 well). Novex Mark 12 molecular weight markers with a mass range from 2.5 to 200 kDa were used for calibration, bovine serum albumin (BSA) was used as sensitivity standard (2 ng). Approximately 1 µg of all the samples and 0.5 µg of standard were applied to the gel. The running conditions were 125 V and 6 W for 120 min. The proteins were fixed and stained using the silver staining kit SilverXpress from Novex.

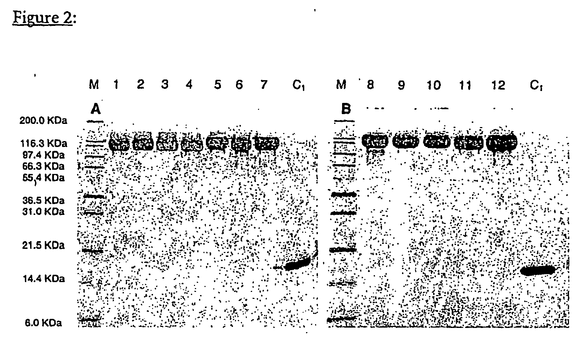

The gels that were recorded under non-reducing conditions for the IEC fractions 1- 8 (Figure 2) show a pattern that is comparable to that of the PEG-IFN alpha 2a reference standard.

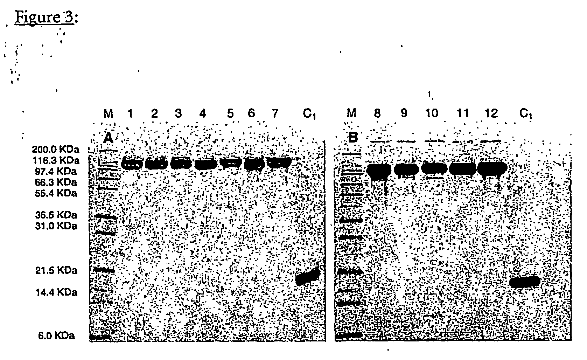

Under reducing conditions, the gels show an increase in intensity of the minor bands at about 90 kDa as compared to the standard. Between 6 and 10 kDa protein fragments appear for peaks 6, 7 and 8 (Figure 3). Both bands together correspond to approximately 1 % of clipped material. In the lanes of isomer 1, 5, 6, 7, 8 additional bands with more than 100 kDa can be seen which are also present in the standard. These can be assigned to oligomers. Thus SDS-PAGE confirms the results of the SE-HPLC analysis.

Overall, RP-HPLC and SDS-PAGE experiments indicate that the purity of the IEC fractions can be considered comparable to the PEG-IFN alpha 2a reference standard.

The structure of the PEG-IFN alpha 2a species derived from the 9 IEC-fractions were identified based on the results of the methods described above using the strategy mentioned above.

### Example 7 The antiviral activity (AVA)

The antiviral activity was estimated by its protective effect on Madin-Darby bovine kidney (MDBK) cells against the infection by vesticular stomatitis virus (VSV) and compared with a PEG-IFN alpha 2a standard. Samples and reference standard were diluted in Eagle's Minimum Essential Medium (MEM) containing 10 % fetal bovine serum to a final concentration of 10 ng/mL (assay starting concentration). Each sample was assayed in quadruplicate.

The antiviral protection of Madin-Darby bovine kidney cells (MDBK) with vesicular stomatitis virus was tested according to the method described in *Virol.* 1981, 37, 755-758. All isomers induced an activity in the anti-viral assay as presented in Table 2. The activities range between 1061 and 339 U/µg, indicating that the difference in specific activities of the protein in the positional isomers is significant. The know-how and the results generated so far will allow the initiation of further investigations to establish this structure-function relationship between the positional isomers and the IFN alpha receptors.

**Table 2:**

| In Vitro Antiviral Activities of PEG-IFN alpha 2a and individual PEG-IFN alpha 2a isomers. The Antiviral activity was determined in MDBK cells infected with vesicular stomatitis virus. The results present the averages of three assays performed independently. | |
|---|---|
| Antiviral Assay of PEG-IFN | |
| Peak | U/µg |
| PEG-IFN | 1061 ± 50 |
| Peak 1 | 1818 ± 127 |
| Peak 2 | 1358 ± 46 |
| Peak 3 | 761197 |
| Peak 4 | 339 ± 33 |
| Peak 4a | 966 ± 107 |
| Peak 5 | 600 ± 27 |
| Peak 6 | 463 ± 25 |
| Peak7 | 513 ± 20 |
| Peak 8 | 468 ± 23 |

The results are further illustrated by the following figures
Figure 1: Analytical IEC-HPLC of 180µg of PEG-IFN alpha 2a. An analytical strong-cation exchange column was used to check the purity of the separated positional isomers from each purification step (TOSOH-BIOSEP, SP-SPW,10 µm particle size, 7.5 mm diameter, 7.5 cm length).
Figure 2: A/B: SDS-PAGE analysis with Tris-glycine (16%), the samples were electrophoresed under non-reduced conditions. The gels were stained for protein with Silver Stain. Lanes: M, molecular weight marker proteins/ 2, Peak 1/ 3, Peak 2/ 4, Peak 3/ 5, Peak 4/ 6, Peak 4a/ 7, Peak 5/ 8, Peak 6/ 9, Peak 7/10, Peak 8/ 11, Ix PEG-IFN standard/ 12, 1.5x PEG-IFN standard/ C₁, IFN standard.
Figure 3: A/B: SDS-PAGE analysis with Tris-glycine (16%), the samples were electrophoresed under reduced conditions. The gels were stained for protein with Silver Stain. Lanes: M, molecular weight marker proteins/ 2, Peak 1/ 3, Peak 2/ 4, Peak 3/ 5, Peak 4/ 6, Peak 4a/ 7, Peak 5/ 8, Peak 6/ 9, Peak 7/ 10, Peak 8/ 11, 1x PEG-IFN standard/ 12, 1.5x PEG-IFN standard/ C₁, IFN standard.
Figure 4: Size Exclusion (SE-) HPLC was used to determine the amount of oligo PEG-IFN forms and aggregates in the different IEC fractions. SE-HPLC was performed with a TosoHaas TSK gel G 4000 SWXL column (7.8 × 300 mm).
Figure 5: MALDI-TOF spectrometry was used to determine the molecular weight of each isomer in order to ensure that the PEG-IFN molecules were still intact after IEC chromatography and to confirm the monopegylation.
Figure 6: MALDI-TOF Lys-C peptide maps of the PEG-IFN reference standard and the peaks 1, 2, 3, 4, 4a, 5, 6, 7, 8. Missing peaks compared to the standard are indicated by arrows.
Figure 7: RP-HPLC chromatograms of the Lys-C digests of the PEG-IFN reference and peak 4a
Figure 8: Analytical HPLC of 5-10µg of PEG-IFN alpha 2a mixture of positional isomers on a column charged with SP-NPR, TosoH Bioscience, Particle size: 2.5µm, nonporous as described in Example 1B..
Figure 9: Ribbon structure of interferon alpha-2a showing the pegylation sites. This is the high resolution structure of human interferon alpha-2a determined with NMR spectroscopy see *J*. *Mol. Biol.* 1997, 274, 661-675. The pegylation sites of pegylated interferon alpha-2a are coloured red and labelled with residue type and residue number.

## Claims

1. The positional isomers of pegylated interferon alpha 2a selected from PEG-Lys(31), PEG-Lys(134), wherein the average molecular weight of the polyethylene glycol moiety (PEG moiety) in said pegylated interferon is from 26,000 daltons to 66,000 daltons, especially of about 40000 daltons.

2. A method for the isolation of the positional isomers of claim 1, **characterised in that** the pegylated interferon is
a) separated into its positional isomers on a preparative liquid chromatography column with a weak-cation exchange matrix; and
b) the fractions are further separated and purified on a preparative column with a strong-cation exchange matrix.

3. A method for the isolation of positional isomers of Lys-pegylated interferon alpha 2a, **characterised in that** the pegylated interferon is
a) separated into its positional isomers on a preparative liquid chromatography column with a weak-cation exchange matrix; and
b) the fractions are further separated and purified on a preparative column with a strong-cation exchange matrix, wherein the average molecular weight of the polyethylene glycol moiety (PEG moiety) in said pegylated interferon is from 26,000 daltons to 66,000 daltons.

4. The method of claim 2 or 3, wherein the chromatographic step a) is conducted by applying a linear pH gradient from about pH 3,8 to pH 8.0, of increasing sodium acetate concentration.

5. The method according to any one of claims 2 to 4, **characterised in that** the chromatographic step b) is conducted with linear gradient of a sodium acetate buffer (A) to a potassium phosphate buffer (B) starting from an initial pH 4.2 to about 4.6 to a final pH of about pH 6.4 to about 6.8, said buffer solutions containing in addition up to 12% ethanol and up to 1.5% diethylene glycol.

6. The method according to claim 5, **characterised in that** the chromatographic steps are carried out at a temperature of about 27° C to about 35° C, preferably at a temperature of about 30 to 32°C.

7. Pharmaceutical compositions comprising a positional isomer of pegylated interferon alpha 2a according to claim 1 and a therapeutically inert carrier.

8. Pharmaceutical compositions for the treatment or prophylaxis of immunomodulatory disorders such as neoplastic diseases or infections diseases comprising a positional isomer of pegylated interferon alpha 2a according to claim 1 and a therapeutically inert carrier.

9. A positional isomer of pegylated interferon alpha 2a according to claim 1 for the manufacturing of medicaments for use in the treatment of illnesses.

10. The positional isomer of claim 9, for the preparation of a medicament for antiproliferative, antiviral and immunomodulatory uses.

11. The positional isomer of claim 10 for the preparation of medicaments for the treatment of viral diseases.

12. The use of a positional isomer of pegylated interferon alpha 2a according to claim 1 for the manufacture of medicaments for use in the treatment or prophylaxis of illnesses.

13. The use of claim 12 for the manufacture of medicaments for antiproliferative, antiviral and immunomodulatory uses.

14. The use of claim 13 for the manufacture of medicaments for the treatment of viral diseases.

## Patentansprüche

1. Stellungsisomere von pegyliertem Interferon alpha-2a, ausgewählt aus PEG-Lys (31), PEG-Lys (134), wobei das durchschnittliche Molekulargewicht der Polyethylenglykoleinheit (PEG-Einheit) in dem pegylierten Interferon 26.000 Dalton bis 66.000 Dalton, insbesondere etwa 40.000 Dalton, beträgt.

2. Verfahren zur Isolierung der Stellungsisomere nach Anspruch 1, **dadurch gekennzeichnet, daß** das pegylierte Interferon
a) in seine Stellungsisomere auf einer präparativen Flüssigchromatographiesäule mit einer schwachen Kationenaustauschmatrix abgetrennt wird; und
b) die Fraktionen weiter abgetrennt und auf einer präparativen Säule mit einer starken Kationenaustauschmatrix gereinigt werden.

3. Verfahren zur Isolierung von Stellungsisomeren von Lys-pegyliertem Interferon alpha-2a, **dadurch gekennzeichnet, daß** das pegylierte Interferon
a) in seine Stellungsisomere auf einer präparativen Flüssigchromatographiesäule mit einer schwachen Kationenaustauschmatrix abgetrennt wird; und
b) die Fraktionen weiter abgetrennt und auf einer präparativen Säule mit einer starken Kationenaustauschmatrix gereinigt werden,
wobei das durchschnittliche Molekulargewicht der Polyethylenglykoleinheit (PEG-Einheit) in dem pegylierten Interferon 26.000 Dalton bis 66.000 Dalton beträgt.

4. Verfahren nach Anspruch 2 oder 3, wobei der Chromatographieschritt a) durch Anwenden eines linearen pH-Gradienten von etwa pH 3,8 bis pH 8,0 zum Erhöhen der Natriumacetatkonzentration durchgeführt wird.

5. Verfahren nach einem der Ansprüche 2 bis 4, **dadurch gekennzeichnet, daß** der Chromatographieschritt b) mit einem linearen Gradienten eines Natriumacetatpuffers (A) zu einem Kaliumphosphatpuffer (B) durchgeführt wird, ausgehend von einem anfänglichen pH 4,2 bis etwa 4,6 bis zu einem End-pH von etwa pH 6,4 bis etwa 6,8, wobei die Pufferlösungen zusätzlich bis zu 12 % Ethanol und bis zu 1,5 % Diethylenglykol enthalten.

6. Verfahren nach Anspruch 5, **dadurch gekennzeichnet, daß** die Chromatographieschritte bei einer Temperatur von etwa 27 °C bis etwa 35 °C, vorzugsweise bei einer Temperatur von etwa 30 bis 32 °C, durchgeführt werden.

7. Pharmazeutische Zusammensetzungen, umfassend ein Stellungsisomer von pegyliertem Interferon alpha-2a nach Anspruch 1 und einen therapeutisch inerten Träger.

8. Pharmazeutische Zusammensetzungen zur Behandlung oder Prophylaxe von Immunomodulationsstörungen, wie neoplastischen Krankheiten oder Infektionskrankheiten, umfassend ein Stellungsisomer von pegyliertem Interferon alpha-2a nach Anspruch 1 und einen therapeutisch inerten Träger.

9. Stellungsisomer von pegyliertem Interferon alpha-2a nach Anspruch 1 zur Herstellung von Medikamenten zur Verwendung bei der Behandlung von Krankheiten.

10. Stellungsisomer nach Anspruch 9 zur Herstellung eines Medikaments für antiproliferative, antivirale und immunomodulatorische Verwendungen.

11. Stellungsisomer nach Anspruch 10 zur Herstellung von Medikamenten für die Behandlung von Viruskrankheiten.

12. Verwendung eines Stellungsisomer von pegyliertem Interferon alpha-2a nach Anspruch 1 zur Herstellung von Medikamenten für die Verwendung bei der Behandlung oder Prophylaxe von Krankheiten.

13. Verwendung nach Anspruch 12 zur Herstellung von Medikamenten für antiproliferative, antivirale und immunomodulatorische Verwendungen.

14. Verwendung nach Anspruch 13 zur Herstellung von Medikamenten für die Behandlung von Viruskrankheiten.

## Revendications

1. Isomères positionnels d'interféron alpha 2a pégylé, choisi parmi PEG-Lys(31), PEG-Lys(134), dans lesquels le poids moléculaire moyen du fragment polyéthylène glycol (fragment PEG) dans ledit interféron pégylé est de 26 000 daltons jusqu'à 66 000 daltons, en particulier d'environ 40 000 daltons.

2. Procédé pour l'isolation des isomères positionnels de la revendication 1, **caractérisé en ce que** l'interféron pégylé est
a) séparé en ses isomères positionnels sur une colonne de chromatographie liquide préparatoire avec une matrice échangeuse de cations faibles ; et
b) les fractions sont de plus séparées et purifiées sur une colonne préparatoire avec une matrice échangeuse de cations forts.

3. Procédé pour l'isolation d'isomères positionnels d'interféron alpha 2 Lys-pégylés, **caractérisé en ce que** l'interféron pégylé est
a) séparé en ses isomères positionnels sur une colonne de chromatographie liquide préparatoire avec une matrice échangeuse de cations faibles ; et
b) les fractions sont, de plus, séparées et purifiées sur une colonne préparatoire avec une matrice échangeuse de cations forts, dans lequel le poids moléculaire moyen du fragment de polyéthylène glycol (fragment PEG) dans ledit interféron pégylé est de 26 000 daltons jusqu'à 66 000 daltons.

4. Procédé selon la revendication 2 ou 3, dans lequel l'étape chromatographique a) est conduite par application d'un gradient de pH linéaire d'environ pH 3,8 jusqu'à pH 8,0 de concentration d'acétate de sodium croissante.

5. Procédé selon l'une quelconque des revendications 2 à 4, **caractérisé en ce que** l'étape chromatographique b) est conduite avec un gradient linéaire d'un tampon d'acétate de sodium (A) sur un tampon de phosphate de potassium (B), en partant d'un pH initial de 4,2 à environ 4,6 jusqu'à un pH final d'environ 6,4 à environ 6,8, lesdites solutions tampon contenant, de plus, jusqu'à 12 % d'éthanol et jusqu'à 1,5 % de diéthylène glycol.

6. Procédé selon la revendication 5, **caractérisé en ce que** les étapes chromatographiques sont effectuées à une température d'environ 27°C jusqu'à environ 35°C, de préférence à une température d'environ 30 à 32°C.

7. Compositions pharmaceutiques comprenant un isomère positionnel d'interféron alpha 2a pégylé selon la revendication 1 et un véhicule thérapeutiquement inerte.

8. Compositions pharmaceutiques pour le traitement ou la prophylaxie de troubles immunomodulateurs, tels que les infections néoplastiques ou maladies infectieuses, comprenant un isomère positionnel d'interféron alpha 2a pégylé selon la revendication 1 et un véhicule thérapeutiquement inerte.

9. Isomère positionnel d'interféron alpha 2a pégylé selon la revendication 1 pour la fabrication de médicaments destinés à l'utilisation dans le traitement de maladies.

10. Isomère positionnel selon la revendication 9 pour la préparation d'un médicament destiné à des utilisations antiprolifératives, antivirales et immuno - modulateurs.

11. Isomère positionnel selon la revendication 10 pour la préparation de médicaments destinés au traitement de maladies virales.

12. Utilisation d'un isomère positionnel d'interféron alpha 2a pégylé selon la revendication 1 pour la fabrication de médicaments destinés à l'utilisation dans le traitement ou la prophylaxie de maladies.

13. Utilisation selon la revendication 12 pour la fabrication de médicaments destinés à des utilisations antiprolifératives, antivirales et immunomodulatrices.

14. Utilisation selon la revendication 13 pour la fabrication de médicaments destinés au traitement de maladies virales.
